(19) **Europäisches Patentamt / European Patent Office / Office européen des brevets**

(11) **EP 3 892 756 A1**

(12) # EUROPEAN PATENT APPLICATION

(43) Date of publication:
**13.10.2021 Bulletin 2021/41**

(51) Int Cl.:
*C23C 24/08* (2006.01)   *B22F 1/00* (2006.01)
*C25B 11/00* (2021.01)   *G01N 27/30* (2006.01)
*H01B 1/20* (2006.01)   *A61B 5/0408* (2006.01)

(21) Application number: **20168542.7**

(22) Date of filing: **07.04.2020**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Heraeus Deutschland GmbH & Co KG
63450 Hanau (DE)**

(72) Inventors:
• **Nikolaidis, Ilias**
  **63450 Hanau (DE)**
• **Schibli, Stefan**
  **63450 Hanau (DE)**
• **Reisinger, Andreas**
  **63450 Hanau (DE)**
• **Gebert, Jörg-Martin**
  **63450 Hanau (DE)**

(74) Representative: **Herzog IP Patentanwalts GmbH
Steinstraße 16-18
40212 Düsseldorf (DE)**

(54) **IMPROVED ELECTROCHEMICAL WIRE COATING**

(57) The invention relates to a method of manufacture of a composition, comprising at least these steps: (i) Providing a precursor formulation which comprises at least silver chloride and an organic vehicle; wherein the precursor formulation comprises agglomerates, preferably of silver chloride particles; and (ii) comminuting the precursor formulation, wherein the composition is obtained. The invention further relates to a substrate which is at least partially coated with a coating comprising silver chloride, wherein the coating has an average size of agglomerates of ... μm or less, to an electrochemical sensor comprising at least one of these coated substrates and a use of the composition or a wire which is coated with the composition.

**Figure 3**

EP 3 892 756 A1

**Description**

The invention relates to a method of manufacture of a composition, comprising at least these steps: (i) Providing a precursor formulation which comprises at least silver chloride and an organic vehicle; wherein the precursor formulation comprises agglomerates, preferably of silver chloride particles; and (ii) comminuting the precursor formulation, wherein the composition is obtained. The invention further relates to a substrate which is at least partially coated with a coating comprising silver chloride, wherein the coating has an average size of agglomerates of 30 $\mu$m or less, to an electrochemical sensor comprising at least one of these coated substrates and a use of the composition or a wire which is coated with the composition.

Electrodes comprising a layer of silver chloride are well known. The basic setup of such electrodes is a conductive core, such as silver, platinum, and the like, which is covered by a layer of silver chloride at the sensing end of the electrode. Such electrodes are often used as reference electrodes, i.e. the electrode is immersed in a liquid and encapsulated by a separating element, e.g. a glass tube or plastic tube. The separating element contains at least a diaphragm or other kind of membrane which allows some kind of ion exchange between the encapsulated electrode and the surroundings of the encapsulated electrode. Reference electrodes are widely used to measure differences in electrical potential with a second electrode and to determine the pH of a system or the difference in potential ($\Delta$U) between the two electrodes, and further e.g. glucose measurement in human blood and the like.

Though these electrodes are quite common, several limitations are known, e.g. in terms of electrical capacity, electrochemical activity, variations in performance, limited lifetime and/or limited miniaturization of the electrode body. Miniaturization, performance and life time are in particular important with applications in human health care.

In general terms, it is an object of the present invention to at least partly overcome one disadvantage which arises from the prior art.

It is an object of the present invention to provide silver chloride electrodes with improved electrochemical properties.

Another object is to provide silver chloride electrodes with improved capacity.

Another object is to provide silver chloride electrodes with less variation in electrochemical performance when comparing electrodes from the same production batch.

Another object is to provide silver chloride electrodes with less variation in electrochemical performance when comparing different batches of electrodes.

Another object is to provide silver chloride electrodes with improved electrochemical activity.

Another object is to provide silver chloride electrodes with enhanced lifetime.

Another object is to provide silver chloride electrodes with smaller dimensions of the electrode.

Another object is to provide silver chloride electrodes with a smoother surface.

Another object is to provide silver chloride electrodes which cause less turbulences when positioned in a fluid stream, e.g. of circulating blood.

Another object is to provide silver chloride electrodes which cause less damage when implanted or inserted into human tissue.

Another object is to provide silver chloride electrodes having smaller and/or less defects on the surface.

It is another object to provide a method of manufacturing a silver chloride electrode with improved electrochemical properties.

Another object is to provide a method of manufacture of a silver chloride electrode with less variation of the characteristics of the manufactured electrodes.

It is another object to provide a method of manufacturing silver chloride electrodes which have an enhanced lifetime when compared with known silver chloride electrodes.

It is another object to provide a method of manufacturing silver chloride electrodes which have an enhanced capacity when compared with known silver chloride electrodes.

It is another object to provide a method of manufacturing silver chloride electrodes which have a smaller form factor while maintaining the electrode capacity, when compared with conventional silver chloride electrodes.

It is another object to provide a method of manufacturing silver chloride electrodes which have a smoother surface.

It is another object to provide a method of manufacturing silver chloride electrodes which cause less turbulences when positioned in a fluid stream, e.g. of circulating blood.

It is another object to provide a method of manufacturing silver chloride electrodes which cause less damage when implanted or inserted into human tissue.

Another object is to provide a more reliable process for manufacturing silver electrodes.

Another object is to provide a more reliable process for manufacturing silver electrodes which may have one or more of the above-mentioned properties.

Another object is to provide a sensor with enhanced lifetime, e.g. compared with conventional sensors of similar kind.

Another object is to provide a sensor with enhanced capacity, e.g. compared with conventional sensors of similar kind.

Another object is to provide a sensor with same capacity but smaller form factor, when compared with conventional silver chloride sensors.

**EMBODIMENTS**

|1| A method of manufacture of a composition, comprising at least these steps:

(i) Providing a precursor formulation which comprises at least silver chloride and an organic vehicle;
wherein the precursor formulation comprises agglomerates;
(ii) comminuting the precursor formulation, wherein the composition is obtained;
wherein at least 75% of the agglomerates in the composition have a size of 30 $\mu$m or less;
wherein the % are based on the number of all agglomerates in the composition.

|2| The method according to embodiment |1|, wherein the size of the agglomerates is determined according to the fineness of grind (FoG) method

|3| The method according to embodiment |1| or |2|, wherein the comminuting in step (ii) comprises

(ii-1) a mixing;
(ii-2) a milling;
(ii-3) an ultrasonic treatment; or
a combination of two or more thereof.

|4| The method according to embodiment |3|, wherein the comminuting comprises at least a mixing and two milling steps.

|5| A method of manufacture of a composite comprising a substrate and at least one coating comprising silver chloride, wherein the method comprises at least these steps:

i) Providing an electrically conductive substrate having a surface;
ii) Superimposing the surface of the substrate at least in part with a composition comprising silver chloride and an organic vehicle thereby forming a layer;
Wherein at least 75% of the agglomerates in the composition have a size or 30 $\mu$m or less;
Wherein the % are based on the number of all agglomerates in the composition;
iii) solidifying the layer.

|6| The method of embodiment |5|, wherein the composition comprising silver chloride and an organic vehicle is obtained according to the method of any one of embodiments |1| to |4|.

|7| The method according to embodiment |5| or |6|, wherein at least 75% of the silver chloride agglomerates in the coating have a size of 30 $\mu$m or less, wherein the % are based on the number of all agglomerates in the coating.

|8| The method according to any one of the preceding embodiments wherein the superimposing in step ii) comprises a technique selected from the group consisting of die coating, dip coating and screen printing, or a combination of two or more thereof.

|9| A composite comprising a substrate and a coating, wherein the substrate is at least partially coated with a coating comprising silver chloride which is at least in part present as agglomerates in the coating, wherein at least 75 % of the AgCl agglomerates have a size of 30 $\mu$m or less, wherein the % are based on the number of all AgCl agglomerates in the coating.

|10| The composite according to embodiment |9|, obtainable by a method according to any one of embodiments |5| to |8|.

|11| An electrochemical sensor comprising at least a composite obtainable by a method according to any one of embodiments |5| to |8|, or a composite according to embodiment |9| or |10|.

|12| The sensor of embodiment |11|, wherein the sensor has an at least double lifetime compared with a conventional AgCl-electrode.

|13| A use of a composition comprising at least silver chloride and an organic vehicle, wherein the composition comprises agglomerates, wherein at least 75% of the agglomerates have a size of 30 $\mu$m or less for the manufacture of a composite which comprises a substrate which is at least partially covered by a layer of silver chloride.

|14| A use of a composition comprising at least silver chloride and an organic vehicle, wherein the composition comprises agglomerates of silver chloride, wherein at least 75% of the agglomerates have a size of 30 $\mu$m or less for the manufacture of an electrochemical sensor wherein the electrochemical sensor has an improved reference capacity, electrochemical activity, improved lifetime, an at least doubled lifetime when compared with conventional AgCl electrochemical sensors.

|15| A use of a composite comprising a wire coated at least partially with a coating of silver chloride wherein at least 75 % of the silver chloride agglomerates in the coating have a size of 30 $\mu$m or less for the manufacture of sensors and devices to determine the blood sugar concentration with improved sensitivity, electrochemical sensors of enhanced lifetime, and/or smaller electrochemical sensor.

## GENERAL

Ranges mentioned in this description also include the values specified as limits. A range of the type "in the range from X to Y" in relation to a size A therefore means that A can assume the values X, Y and values between X and Y. Single sided limited ranges of the type "up to Y" for a size A are correspondingly interpreted as values Y and smaller than Y.

## DESCRIPTION OF THE INVENTION

A contribution to at least partial achievement of at least one of the above objects is made by the independent claims. The dependent claims provide preferred embodiments which contribute to at least partial achievement of at least one of the objects.

A first aspect is a method of manufacture of a composition, comprising at least these steps:

(i) Providing a precursor formulation which comprises at least silver chloride and an organic vehicle; wherein the precursor formulation comprises agglomerates;
(ii) comminuting the precursor formulation, wherein the composition is obtained; wherein at least 75% of the agglomerates in the composition have a size of 30 $\mu$m or less; wherein the % are based on the number of all agglomerates in the composition.

Step (i) providing can be achieved by any kind which is known and considered suited by a skilled person. More specifically, it is contemplated to put an organic vehicle and silver chloride particles in a mixing device. As a result, a precursor formulation is obtained.
The organic vehicle can be of any kind known and considered suited by a skilled person. More specifically, the organic vehicle may comprise one or more organic solvents and, optionally, one or more additives.
Organic solvents that suit in the present invention are in general known to the skilled person. Preferred types of organic solvents suited in the present invention are non-polar, polar aprotic or polar protic solvents, for example toluene, terpineol, texanol, isopropyl alcohol, ethyl acetate or a combination of two or more thereof.
Silver chloride can be added in any configuration considered suited by a skilled person. For example, silver chloride can be used in particle form. Preferred particle sizes of silver chloride are in the range from 1 $\mu$m to 5 $\mu$m. The particles may also be present in form of agglomerates.
Agglomerates are in the present context are clusters which usually form by aggregation of a plurality of particles. Agglomerates can form to sizes of tens to hundreds $\mu$m and even more.
The agglomerates are predominantly formed by silver chloride particles. Minor amounts of other particles, such as silver, dirt, etc. can be incorporated. Usually, the agglomerates contain only silver chloride particles. Moreover, the agglomerates may contain molecules of the organic vehicle.
In a second step, step (ii), the precursor formulation which comprises at least silver chloride and an organic vehicle is comminuted whereby the composition is obtained. At least 75%, e.g. 80% or 90% of the agglomerates in the composition after comminution have a size of 30 $\mu$m or less. For example, the size of agglomerates in the composition 20 $\mu$m or less, 15 $\mu$m or less, 10 $\mu$m or less, or even 5 $\mu$m or less. Usually at least some agglomerates have a size of

at least 1 $\mu$m.

When referring to the size of agglomerates, it is meant that at least 75%, e.g. at least 80%, or at least 90%, or at least 95% of the agglomerates have the indicated size. Often, not more than 99% of the agglomerates have the indicated size. The remainder may have sizes that vary.

However, the variation can be limited, i.e. another 20% of the agglomerates have a size which is up to 10% above of the referred-to size. Less than 5% of the agglomerates may have a size that is more than $\pm$10% above of the referred-to size. For example, if the size if agglomerates is described as 25 $\mu$m or less, more than 75% of the agglomerates have an agglomerates' size of 25 $\mu$m or less, up to 20 % may have agglomerates of a size in the range from 27.5 $\mu$m to more than 25 $\mu$m, and up to 5% may have an agglomerates of a size of more than 27,5 $\mu$m. The values in % of agglomerates are always based on the total number of agglomerates found in a sample of the precursor formulation. The indication of agglomerates' size refers to the longest end-to-end distance measured by a continuous straight connection within a single agglomerate. These values are determined by the so-called fineness of grind method (FoG) which is described in the section "test methods" later on.

In a preferred embodiment, about at least 95% of the silver chloride agglomerates in the composition have a size of 10$\mu$m or less.

The step of comminuting can be performed by any technique known to and considered suited by a skilled person. This step can be comprised one or more different tasks, for example:

(ii-1) a mixing, e.g. speed mixing;
(ii-2) a milling, e.g. ball milling and 3-roll milling;
(ii-3) an ultrasonic treatment; or
a combination of two or more of the above tasks.

A preferred way to perform the comminuting of step (ii) can be a combination of a speed mixing and two milling tasks in a sequence, or a combination of a speed mixing and 3, 4, 5, 6 or 7 milling steps in a sequence.

The composition may be composed of silver chloride, organic vehicle and further components as follows:

5 to 75 wt.%; e.g. 20 to 50 wt.% silver chloride;
0 to 80 wt.%; e.g. 50 to 75 wt.% further components;
Remainder to 100 wt.%: organic vehicle,
wherein all wt.-% are with respect to the total weight of the composition and wherein the sum of all wt.% is always 100wt.%.

The composition may contain further components. These further components can be provided similarly like the silver chloride and the organic vehicle. At least some of the further may also results from abrasion from the mixing and milling vessels used in the comminuting step (ii).

The further components can be of any kind known to and/or considered suited by a skilled person. Examples of the further components include metal particles such as: silver, and ceramic particles such as those comprising aluminium oxide.

A second aspect is a method of manufacture of a composite comprising a substrate and at least a first coating, wherein the first coating comprises silver chloride. The method comprises at least these steps:

i) Providing an electrically conducting substrate, e.g. a metal wire, having a surface;
ii) Superimposing the surface of the substrate at least in part with a composition comprising silver chloride and an organic vehicle;

wherein at least 75%, or at least 80%, or at least 90%, or at least 95% of the agglomerates in the composition have a size or 30 $\mu$m or less, for example 20 $\mu$m or less, or 15 $\mu$m or less, or 5 $\mu$m or less; wherein the % are based on the number of all agglomerates in the composition.

The composition mentioned with this aspect can be the same as the one in the first aspect.
The coating may comprise a mixture of silver and silver chloride.

The substrate can be any such known to and considered suited by a skilled person. Examples of such a substrate are: wire, plate, sheet, with preference a wire.

The substrate can be made from any material known to and considered suited by a skilled person for making electrodes. The substrate is electrically conductive by preference. Electrically conductive in the context of the present invention stands for materials which have an electric conductivity of >= $10^6$ S/m at 25 °C. Examples materials suited in the present invention are silver, platinum, platinum alloy, or a combination of two or more thereto.

The substrate itself can further be a composite of multiple compounds, for example a sheet of polyimide having sputtered platinum on the surface, or a conductive wire which has a layer of insulating material on its surface.

The composite, which is obtained by the method according to the second aspect, can have at least a further coating of insulating material on the silver chloride layer. Any kind of insulating layer can be employed known to and considered possibly suited by a skilled person. Examples of such insulating layers are polyurethane, polyimide, silicone and PTFE.

The first coating may form a distinct pattern on the substrate, wherein features of the pattern are separated by gaps in the coating.

In another embodiment, about at least 75%, or at least 80%, or at least 90%, or at least 95% of the silver chloride agglomerates in the coating have a size of 30 $\mu$m or less, for example 20 $\mu$m or less, or 15 $\mu$m or less, or 5 $\mu$m or less. The % are based on the number of all agglomerates in the coating. The size of the silver chloride agglomerates can be determined using ImageJ, Version 1.52, from the Wayne Rasband National Institutes of Health, USA).

About at least 95% of the silver chloride agglomerates in the coating have a size of agglomerates of 10$\mu$m or less in a preferred embodiment.

The coating may be superimposed in step ii) using a technique selected from the group consisting of die coating, dip coating and screen printing, or a combination of two or more thereof.

After superimposing the coating onto the surface of the substrate, the coating is solidified on the substrate. Solidification of the liquid constituents of the coated composition can occur either by

- removal of a constituent or
- by reaction of one or more constituents or
- by reaction of a constituent with functional groups of the substrate,
- or by a combination of two or more of these mechanisms.

Any means and techniques, known to a skilled person and considered apt in the present method can be employed to achieve solidification of the coating. For example, evaporation, a thermal treatment, e.g. with heat, treatment with irradiation, e.g. microwaves, exposure to reduced pressure or vacuum etc. appear suited. Some examples of the liquid constituents' solidification due to a chemical reaction are cross-linking, gelation and polymerization, or combinations thereof.

In a third aspect is provided a composite obtainable by a method according to the second aspect.

A fourth aspect is a composite comprising a substrate and a coating, wherein the substrate is at least partially coated with a coating, wherein the coating comprises at least silver chloride which might be present in form of agglomerates, wherein at least 95% of the agglomerates have a size of 10$\mu$m or less. Preferred embodiments with regard to the silver chloride, the substrate, the coating and the agglomerates are identical with the ones described above. The composite may have at least one of the features of those described for the composite of the second aspect, or a combination of two or more thereof.

A fifth aspect is an electrochemical sensor which may be implantable or inserted into a human and/or comprise at least an electrode, wherein the sensor, or the electrode, or both, comprise at least a composite obtainable by a method as described in the second aspect or a composite as described third or fourth aspect. In a preferred embodiment, the electrochemical sensor has a doubled lifetime compared with conventional AgCl-electrodes.

This sensor can be of any kind known to and considered suited by a skilled person for use with a silver chloride electrode. Examples are electrodes for pH-meters and blood glucose concentration.

A sixth aspect is a use of a composition comprising at least silver chloride and an organic vehicle, wherein the composition comprises agglomerates, e.g. as described above, wherein at least 75%, or at least 80%, or at least 90%, or at least 95% of the agglomerates have a size of 30 $\mu$m or less, or 20 $\mu$m or less, or 15 $\mu$m or less, or 10 $\mu$m or less, or 5 $\mu$m or less for the manufacture of a composite which comprises a substrate which is at least partially covered by a layer of silver chloride. Embodiments and modifications described above are included by reference.

An seventh aspect is a use of a composition comprising at least silver chloride and an organic vehicle, wherein the composition comprises agglomerates of silver chloride, wherein at least 75%, or at least 80%, or at least 90%, or at least 95% of the agglomerates have a size of 30 $\mu$m or less, or 20 $\mu$m or less, or 15 $\mu$m or less, or 10 $\mu$m or less, or 5 $\mu$m or less for the manufacture of an electrochemical sensor, e.g. an electrode, wherein the electrochemical sensor has an improved reference capacity, electrochemical activity, improved lifetime, higher accuracy , an at least doubled lifetime when compared with conventional AgCl electrochemical sensors. Embodiments and modifications described above are included by reference.

A eighth aspect is a composite comprising a wire coated at least partially with a coating of silver chloride, wherein at least 75 % of the silver chloride agglomerates in the coating have a size of 30 $\mu$m or less for the manufacture of sensors and devices to determine the blood sugar concentration with improved sensitivity, electrochemical sensors of enhanced lifetime, and/or smaller electrochemical sensor. Blood sugar concentration can be determined by measuring the glucose and/or fructose concentration of blood, Embodiments and modifications described above are included by reference.

## LIST OF FIGURES

Figure 1 is a schematic representation of a method of manufacture of a composition.
Figure 2 is a schematic representation of a method of manufacture of a composite.
Figure 3 is a schematic cross-sectional view of a composite.
Figure 4 is a schematic top view of a sensor.
Figure 5 shows a schematic representation of the experimental setup in chronopotentiometry.
Figure 6 shows a schematic drawing of a working electrode with a AgCl layer for testing.
Figure 7 shows three datasets obtained from measurements using the setup of Figure 5.
Figure 8A shows a schematic sideview of sample having a substrate having a polymer matrix coating, where the AgCl particles were used as is and deposed from dispersion to the substrate.
Figure 8B shows a similar view for a similar sample, where the AgCl in the coating layer was comminuted prior to application to the substrate so that at least 75% of any agglomerates of AgCl in the composition had a size of 30 $\mu$m or less.

## DESCRIPTION OF THE FIGURES

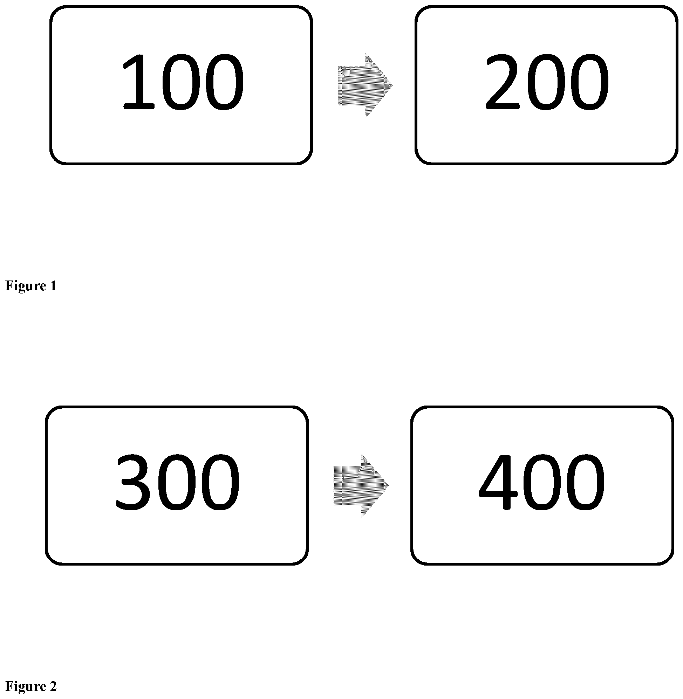

Figure 1 is a schematic representation of a method of manufacture of a composition. In step (i), a precursor formulation is provided which comprises at least silver chloride and an organic vehicle; wherein the precursor formulation may comprise agglomerates. In step (ii), the precursor formulation is comminuted. After comminution, at least 75% of the agglomerates in the composition have a size of 30 $\mu$m or less, the % based on the number of all agglomerates in the composition.

Figure 2 is a schematic representation of a method of manufacture of a composite comprising a substrate and at least a coating comprising silver chloride. In step i), an electrically conductive substrate having a surface is provided. In step ii), the surface of the substrate is at least in part coated with a composition comprising silver chloride and an organic vehicle. In this composition, at least 75% of the agglomerates have a size or 30 $\mu$m or less, the % based on the number of all agglomerates in the composition. Liquid parts of the composition, for example, the organic vehicle, are evaporated afterwards so that the coating solidifies.

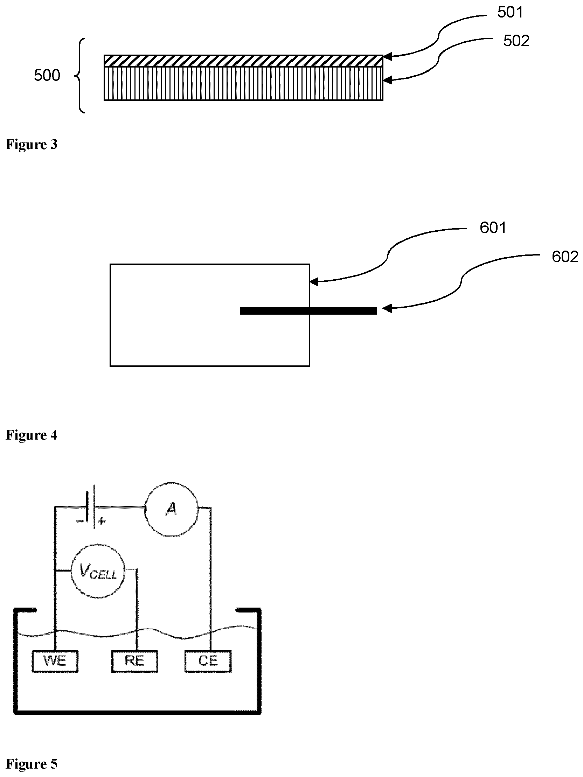

Figure 3 is a schematic cross-sectional view of a composite. The composite comprises a substrate which is covered by and in contact with a top coat.

Figure 4 is a schematic top view of a sensor. The sensor includes a composite as in Figure 3. The composite may protrude over the sensor body.

Figure 5 shows a schematic representation of the experimental setup in chronopotentiometry. Three electrodes, a working electrode (WE), a reference electrode (RE) and a counter electrode (CE) are positioned submerged in a beaker in a electrolyte, a standard phosphate-buffered saline (PBS) solution. A constant current, e.g. 1 $\mu$A, is applied between the working electrode and the counter electrode. The reference electrode is connected to the working electrode through a voltmeter which measures the voltage $V_{cell}$ between the electrodes. During an initial 5-6 minutes of a measurement, the voltage $V_{cell}$ was measured, averaged and recorded as baseline voltage Vo. Then, the time T was recorded until $V_{cell}$ dropped by -50 mV. This time correlates with the lifetime of a sensor in that the higher time T the longer the lifetime of a sensor comprising the working electrode.

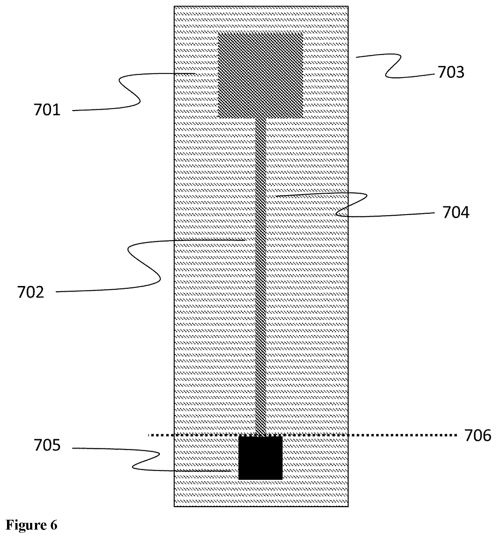

Figure 6 shows a drawing of a working electrode. A substrate, e.g. Kapton, 30 mm x 100 xx x 0.1 mm) is partially covered by line-shaped conducting path, e.g. made of a platinum layer (thickness 0.1 $\mu$m). The conducting path connects two contact areas. One contact area is covered with a layer of AgCl, prepared according to the invention, or with a layer of another material for comparison. The other contact area is designed to be connected to measuring devices and/or other circuitry.

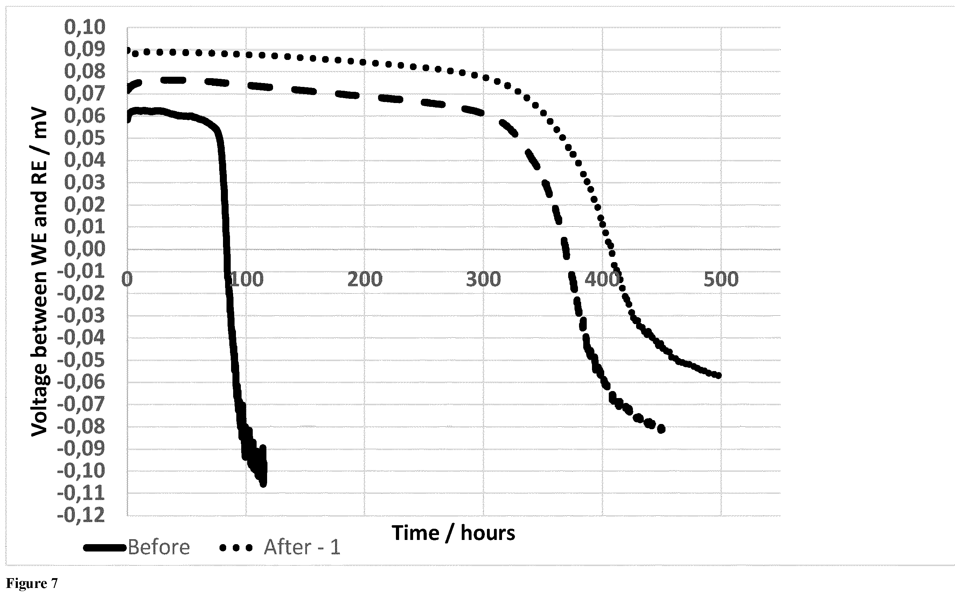

Figure 7 shows three datasets obtained from measurements using the setup of Figure 5. The solid line labelled "0" represents an Ag/AgCl electrode which has no coating. The dashed line labelled "1" and the dotted line labelled "2" were recorded when testing with working electrode which had a coating comprising silver chloride which were at least in part present as agglomerates in the coating. 95±5 % of the AgCl agglomerates/particles had a size of 3 $\mu$m or less.

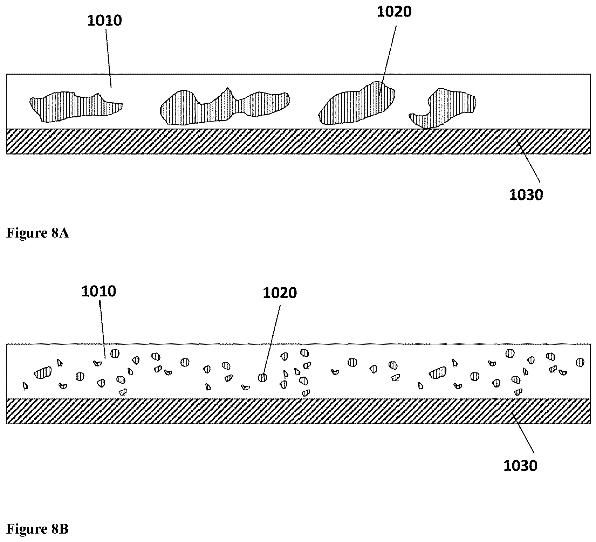

Figure 8A shows a schematic sideview of sample having a substrate having a polymer matrix coating, where the AgCl particles were used as is and deposed from dispersion to the substrate.

Figure 8B shows a similar view for a similar sample, where the AgCl in the coating layer was comminuted prior to application to the substrate so that at least 75% of any agglomerates of AgCl in the composition had a size of 30 $\mu$m or less. The drawings of Fig. 7A and 7B are actual overlays of SEM microstructures and illustrate real data.

**TEST METHODS**

The following test methods were used for the purposes of the invention. Unless otherwise stated the measurements were made at ambient temperature 25°C, ambient air pressure 100 kPa (0.986 atm) and relative humidity 50%.

### a. Fineness of Grind (FoG) - size of agglomerates

Fineness of grind of agglomerates was determined according to DIN EN ISO 1524:2013-06. A gauge for maximum depth of groove of $25\mu m$ having an Interval of graduations of $2,5\mu m$ was used. The procedures for taking samples as described in ISO 15528:2013 were precisely observed under consideration of the physical nature of the material to be tested, as well as ISO 1513 of 2010 regarding the examination and preparation of test samples. No dilution of the sample was necessary for performing this measurement.

### b. Number / percentage of silver chloride agglomerates

Test specimen were prepared by preparing cross-cuts through samples, whereby the cross-cut was made perpendicular to the layer and the substrate. The test specimen was then analysed using Scanning Electron Microscopy (Jeol JSM-6610LV) and graphical analysis (ImageJ 1.52p) which creates overlays representing agglomerates. A sample overlay is shown in Figures 7A and 7B.

### c. Electrode capacity & Electrode lifetime

Chronopotentiometry was used to determine the capacity of an electrode and its lifetime. In this technique, the instrument operates in galvanostatic mode to control current and measure voltage. The applied current is constant at $1\mu A$.

This procedure was used to determine the reference electrode capacity of Ag/AgCl coated sensors. Chronopotentiometry was also used to challenge electrodes with various cathodic currents, thus simulating the intended use life, i.e. days of normal operation. During testing, the silver chloride component of an electrode may be reduced to silver and chloride, as shown in the following equation.

$$AgCl + e^- \rightarrow Ag^0 + Cl^-$$

A standard 3 electrode setup was used for this measurement as shown in Figure 5, whereby:

A REF321 Reference Electrode (Radiometer analytical SAS) was used as reference electrode (RE). The working electrode (WE) was the Ag/AgCl coated sensor element. The coating thickness and surface area of the WE measured were always kept the same so that results are comparable. The counter electrode (CE) was a Pt mesh of at least $1cm^2$ surface area. The measurements were performed with all three electrodes immersed in a 0,5Lt beaker filled with a standard Phosphate-buffered saline (PBS) solution. A constant current (A) of $1\mu A$ was applied between the WE and CE.

A "baseline" voltage" $V_0$ was determined by calculating the average voltage between the RE and WE ($V_{cell}$) based on recording the voltage for 5 to 6 minutes. Then, a time (T) was measured, until $V_{cell}$ dropped by 50mV with respect to the baseline voltage. The Reference Capacity (C) of the AgCl coated sensor was calculated according to:

$$C = T * A,$$

where A was set constant at $1\mu A$, according to the experimental conditions.

The Reference Capacity value is indicative of the lifetime of the sensor. Therefore, the higher this value is the longer such a sensor can produce a meaningful measurement.

**EXAMPLES**

The invention is described in more detail below via Examples and drawings, wherein the Examples and drawings do not imply any restriction of the invention. The drawings are moreover diagrammatic and not true to scale.

### Preparation of a working electrode (invention)

A Kapton sheet of 30 mm x 100 mm and 0.10mm thickness was used as a substrate. A platinum layer of 0. $10\mu m \pm 0.01\ \mu m$ thickness was sputtered through a mask on the Kapton using an ATC-Orion 8 HV machine (AJA International, Inc). The mask was used to create the design of a conducting feature on the substrate (see Figure 6), thereby hiding all other parts of the Kapton substrate.

Then, a pattern of AgCl was created by applying AgCl paste through a nylon 420mesh screen to the Kapton substrate at end of the sputtered platinum conductor path. The AgCl pattern covered a surface of 1x1 mm$^2$ on the Kapton substrate and had a thickness of $40\mu m \pm 10\mu m$. On the other end of the conductive platinum feature is a larger pad, which is used to connect the working electrode to measuring equipment. The Kapton electrode with the printed film was cured at 200°C for 20 minutes. The resulting printed AgCl layer had a thickness of $25 \pm 2,5\mu m$.

Preparation of a working electrode (comparative)

A Kapton sheet with a conducting feature was prepared as described above. Then, a pattern of a comparative material was applied in a similar manner. Finally, the Kapton electrode with the coated pattern was cured at 200°C for 20 minutes. In the event, the coating would not withstand such thermal treatment, lower temperature could be used. The thickness of the coating of the comparative material on the substrate was adjusted in order to obtain a $25 \pm 2,5 \mu m$ thick AgCl layer after the thermal treatment.

Determining the parameters of the working electrode

The part of the Kapton sheet was immersed in an electrolyte liquid so that the area of the AgCl pattern was immersed and chronopotentiometry was conducted. The larger pad of the electrode was connected with the electrical measuring equipment, a Gamry instruments Reference 600 Potentiostat/Galvanostat/ZRA and according to the electrical circuit shown in Figure 5. The measurement was conducted by applying a constant current of 1.0 $\mu A$ between the working electrode and a counter electrode. The voltage between the working electrode (WE) and counter electrode (CE) was recorded over time. Exemplary charts are shown in Figure 7. The test was stopped when the voltage between the working electrode and the counter electrode was dropped by 50mV with respect to the baseline (initial) voltage measured between them (stop condition). The electrode lifetime is determined, by the amount of time required to reach this stop condition. This measurement was conducted for 7 working electrodes of the same specification and the resulting values were averaged using the arithmetic mean.

**REFERENCE NUMERALS**

| | |
|---|---|
| 100 | Step (i) |
| 200 | Step (ii) |
| 300 | Step i) |
| 400 | Step ii) |
| 500 | Composite |
| 501 | Coating |
| 502 | Substrate |
| 601 | Sensor |
| 602 | Composite |
| 701 | Larger pad (to be used for connecting electrode to measuring equipment) |
| 702 | Conductor path for transferring the signal |
| 703 | Kapton substrate |
| 704 | Sputtered Platin layer |
| 705 | 1 x 1 mm printed AgCl paste pad. Screen printed on top of the sputtered Pt. Area to be inside the electrochemical solution during testing. |
| 706 | Level of test solution |
| 1010 | Polymer matrix of coating |
| 1020 | AgCl particles / agglomerates |
| 1030 | Substrate |

**Claims**

1. A method of manufacture of a composition, comprising at least these steps:

(i) Providing a precursor formulation which comprises at least silver chloride and an organic vehicle;

wherein the precursor formulation comprises agglomerates;
(ii) comminuting the precursor formulation, wherein the composition is obtained;

  w herein at least 75% of the agglomerates in the composition have a size of 30 $\mu$m or less;
  w herein the % are based on the number of all agglomerates in the composition.

2. The method according to claim 1, wherein the size of the agglomerates is determined according to the fineness of grind (FoG) method

3. The method according to claim 1 or 2, wherein the comminuting in step (ii) comprises

  (ii-1) a mixing;
  (ii-2) a milling;
  (ii-3) an ultrasonic treatment; or

a combination of two or more thereof.

4. The method according to claim 3, wherein the comminuting comprises at least a mixing and two milling steps.

5. A method of manufacture of a composite comprising a substrate and at least one coating comprising silver chloride, wherein the method comprises at least these steps:

  i) Providing an electrically conductive substrate having a surface;
  ii) Superimposing the surface of the substrate at least in part with a composition comprising silver chloride and an organic vehicle thereby forming a layer;
  Wherein at least 75% of the agglomerates in the composition have a size or 30 $\mu$m or less;
  Wherein the % are based on the number of all agglomerates in the composition;
  iii) solidifying the layer.

6. The method of claim 5, wherein the composition comprising silver chloride and an organic vehicle is obtained according to the method of any one of claims 1 to 4.

7. The method according to claim 5 or 6, wherein at least 75% of the silver chloride agglomerates in the coating have a size of 30 $\mu$m or less, wherein the % are based on the number of all agglomerates in the coating.

8. The method according to any one of the preceding claims wherein the superimposing in step ii) comprises a technique selected from the group consisting of die coating, dip coating and screen printing, or a combination of two or more thereof.

9. A composite comprising a substrate and a coating, wherein the substrate is at least partially coated with a coating comprising silver chloride which is at least in part present as agglomerates in the coating, wherein at least 75 % of the AgCl agglomerates have a size of 30 $\mu$m or less, wherein the % are based on the number of all AgCl agglomerates in the coating.

10. The composite according to claim 9, obtainable by a method according to any one of claims 5 to 8.

11. An electrochemical sensor comprising at least a composite obtainable by a method according to any one of claims 5 to 8, or a composite according to claim 9 or 10.

12. The sensor of claim 11, wherein the sensor has an at least double lifetime compared with a conventional AgCl-electrode.

13. A use of a composition comprising at least silver chloride and an organic vehicle, wherein the composition comprises agglomerates, wherein at least 75% of the agglomerates have a size of 30 $\mu$m or less for the manufacture of a composite which comprises a substrate which is at least partially covered by a layer of silver chloride.

14. A use of a composition comprising at least silver chloride and an organic vehicle, wherein the composition comprises agglomerates of silver chloride, wherein at least 75% of the agglomerates have a size of 30 $\mu$m or less for the manufacture of an electrochemical sensor wherein the electrochemical sensor has an improved reference capacity, electrochemical activity, improved lifetime, an at least doubled lifetime when compared with conventional AgCl electrochemical sensors.

15. A use of a composite comprising a wire coated at least partially with a coating of silver chloride wherein at least 75 % of the silver chloride agglomerates in the coating have a size of 30 $\mu$m or less for the manufacture of sensors and devices to determine the blood sugar concentration with improved sensitivity, electrochemical sensors of enhanced lifetime, and/or smaller electrochemical sensor.

100 ➡ 200

**Figure 1**

300 ➡ 400

**Figure 2**

501
502

500

Figure 3

601
602

Figure 4

WE    RE    CE

Figure 5

701

702

703

704

705

706

Figure 6

Figure 7

EP 3 892 756 A1

**1010**

**1020**

**1030**

Figure 8A

**1010**

**1020**

**1030**

Figure 8B

Europäisches
Patentamt

European
Patent Office

Office européen
des brevets

## EUROPEAN SEARCH REPORT

Application Number

EP 20 16 8542

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 051 208 A (BOWNS RICHARD E [US] ET AL) 24 September 1991 (1991-09-24) * column 1, lines 5-9; example 1 * * column 1, lines 11-21 * * column 1, line 67 - column 3, line 36 * ----- | 1-3,9-14 | INV. C23C24/08 B22F1/00 C25B11/00 G01N27/30 H01B1/20 A61B5/0408 |
| X | US 5 851 438 A (CHAN MAN-SHEUNG [US]) 22 December 1998 (1998-12-22) * column 4, lines 11-52; examples 1, 2, 6 * * column 5, lines 45-52 * * column 6, lines 1-35 * * column 9, line 45 - column 10, line 6 * ----- | 1-14 | |
| X | CN 109 645 986 A (KUNMING INST PRECIOUS METALS) 19 April 2019 (2019-04-19) * paragraph [0001] - paragraph [0002]; claim 6; example 1 * * paragraph [0016] - paragraph [0027] * ----- | 1,3,4,9, 11,13-15 | |

TECHNICAL FIELDS
SEARCHED      (IPC)

C23C
C25B
H05B
C22C
G01N
B22F
H01B
A61B

The present search report has been drawn up for all claims

| Place of search | Date of completion of the search | Examiner |
|---|---|---|
| The Hague | 14 September 2020 | Telias, Gabriela |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                                     
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 16 8542

This annex lists the patent family members relating to the patent documents cited in  the above-mentioned European search report.
The members are as contained in the European Patent Office EDP file on
The European Patent Office is in no way liable for these particulars which are merely given for the purpose of information.

14-09-2020

| Patent document cited in search report | | Publication date | Patent family member(s) | | Publication date |
|---|---|---|---|---|---|
| US 5051208 | A | 24-09-1991 | NONE | | |
| US 5851438 | A | 22-12-1998 | CN | 1211592 A | 24-03-1999 |
| | | | EP | 0899748 A2 | 03-03-1999 |
| | | | JP | 3088704 B2 | 18-09-2000 |
| | | | JP | H11232930 A | 27-08-1999 |
| | | | KR | 19990023981 A | 25-03-1999 |
| | | | TW | 561496 B | 11-11-2003 |
| | | | US | 5851438 A | 22-12-1998 |
| | | | US | 5928571 A | 27-07-1999 |
| CN 109645986 | A | 19-04-2019 | NONE | | |

EPO FORM P0459

For more details about this annex : see Official Journal of the European Patent Office, No. 12/82